# EUROPEAN PATENT APPLICATION

(11) **EP 1 839 639 A1**
(43) Date of publication of application: **03.10.2007**
(21) Application number: 06711672.3
(22) Date of filing: 13.01.2006
(51) Int. Cl.: A61K 6/00

(54) **COSMETIC PREPARATION**

(30) Priority: 14.01.2005 JP 2005007548
(71) Applicant: SUMITOMO SEIKA CHEMICALS CO., LTD., Kako-gun, Hyogo 675-0145 (JP)
(72) Inventor: OKUBO, Masayoshi, c/o Dept. Chemical Sci. and Eng., Nada-ku, Kobe-shi, Hyogo 657-8501 (JP); YAMAGUCHI, Kiyoshi, c/o Osaka Head Office, Chuo-ku, Osaka-shi, Osaka 541-0041 (JP); HAMAMOTO, Shigeki, Functional Polymers Res. Lab., Shikama-ku, Himeji-shi, Hyogo 672-8076 (JP)
(74) Representative: Nicholls, Kathryn Margaret
(86) International application number: PCT/JP2006/300387
(87) International publication number: WO 2006/075711

(57) **Abstract**

The present invention provides a cosmetic comprising a spherical resin particle having many recesses on the surface thereof. According to the present invention, a cosmetic which is excellent in spreadability and a touch feeling at application, and an effect such as a moist feeling, a wet feeling, a dry feeling and the like after application, and retention thereof, as well as a cosmetic which has an optical function such as color rendering property and a foggy feeling (wrinkle degradation effect) are provided.

## Description

### Technical field

The present invention relates to a cosmetic, more particularly, a cosmetic excellent in spreadability and a touch feeling at application, and an effect after application, as well as retention thereof.

### Background Art

For the purpose of improving spreadability, a touch feeling, slidability, and uniformity at application, spherical fine particles having a particle diameter of around 0.1 to 50 µm have been previously added to cosmetics such as makeup cosmetics such as foundation, face powder, rouge, eye shadow, eyebrow and the like, body cosmetics such as body powder, baby powder and the like, and skin care products such as lotion, milky lotion and the like.

For example, a cosmetic containing a spherical polyurethane fine powder having an average particle diameter of not more than 30 µm (Patent Literature 1), an acrylic copolymer fine particle for cosmetics obtained by suspension-polymerizing a monomer mixture containing an (meth)acrylate monomer, an ethyl acrylate monomer, and a polyfunctional vinyl monomer (Patent Literature 2), a cosmetic containing a specific polyethylene-resin spherical fine particle (Patent Literature 3), and the like are known.

However, since all of particles blended in these cosmetics are mere spherical particles, they are excellent in spreadability and a touch feeling at application, but they are not necessarily satisfactory in connection with high functionalization and diversification of cosmetics and, therefore, improvement in manifestation of the effect such as a wet feeling, a moist feeling, and a dry feeling after application and retention thereof has been demanded.

[Patent Literature 1]
JP-A No. 5-262622
[Patent Literature 2]
JP-A No. 2001-151626
[Patent Literature 3]
JP-A No. 2002-370920

### Disclosure of the Invention

An object of the present invention is to provide a cosmetic which is excellent in spreadability and a touch feeling at application, and is excellent in manifestation of the effect such as a wet feeling, a moist feeling, and a dry feeling after application, and retention thereof.

The present invention relates to a cosmetic comprising a spherical resin particle having many recesses on the surface thereof.

According to the present invention, a cosmetic which is excellent in spreadability and a touch feeling at application, and the effect after application, and retention thereof can be provided.

Moreover, a cosmetic with optical functions, such as color rendering property and a foggy feeling (wrinkle degradation effect), can be provided.

### Best Mode for Carrying Out the Invention

The spherical resin particle used in the present invention has many recesses on its surface. "Many" means two or more per particle. As a particle having more recesses on the particle surface, a cosmetic which is more excellent in spreadability and a touch feeling at application, and the effect after application, and retention thereof can be obtained.

A volume average particle diameter of the spherical resin particle used in the present invention is preferably 0.1-50 µm, more preferably 0.5-30 µm. When the volume average particle diameter is less than 0.1 µm, there is a possibility that spreadability of the obtained cosmetic at application is deteriorated. When the volume average particle diameter is more than 50 µm, there is also a possibility that a touch feeling of the obtained cosmetic at application is deteriorated.

The above-mentioned many recesses also usually have an approximately circular opening, and recesses having openings of different diameters are present on the particle surface. An average diameter of the opening of recesses is preferably 0.01-1 µm, more preferably 0.05-0.5 µm. When the average diameter of the opening is smaller than 0.01 µm, it is difficult to obtain a desired performance and, when the average diameter is more than 1 µm, there is a possibility that spreadability and a touch feeling of the cosmetic at application is reduced.

The spherical resin particle used in the present invention is preferably a particle with a narrow particle size distribution, more preferably a monodisperse particle. Inter alia, it is desirable that a variation coefficient CV value which is an index of particle size distribution is not more than 20%, preferably not more than 10%, more preferably not more than 5%.

When the CV value is more than 20%, the particle size distribution of particles becomes broader, and there is a possibility that spreadability and a touch feeling upon application of the cosmetic are deteriorated, being not preferable. The variation coefficient CV value is a value shown by the following equation using a volume average particle diameter and a standard deviation value thereof, obtained by measurement of a particle diameter.

Variation coefficient CV value [%] = (standard deviation value/volume average particle diameter) x 100

In the present specification, the volume average particle diameter and the standard deviation thereof are a numerical value obtained by a laser diffraction-type particle size distribution measuring apparatus, and the average diameter of the opening of the recess, and the average number of recesses per particle are a numerical value obtained by image analysis of an electron micrograph.

The spherical resin particle having many recesses on the surface used in the present invention is not particularly limited, but a composite resin particle in which an inner wall portion of the recess and a particle body are composed of different resins is preferably used. Examples of the resins which constitute a recess part and a particle body, respectively, include acrylic acid ester polymers such as polymethyl acrylate, polyethyl acrylate, polypropyl acrylate, polybutyl acrylate, polynormalhexyl acrylate, polycyclohexyl acrylate, poly2-ethylhexyl acrylate, polydodecyl acrylate, polystearyl acrylate and the like; methacrylic acid ester polymers such as polymethyl methacrylate, polyethyl methacrylate, polypropyl methacrylate, polybutyl methacrylate, polynormalhexyl methacrylate, polycyclohexyl methacrylate, poly2-ethylhexyl methacrylate, polydodecyl methacrylate, polystearyl methacrylate and the like; vinyl ester polymers such as polyvinyl acetate, polyvinyl propionate, and the like; aromatic vinyl polymers such as polystyrene, polyvinyltoluene and the like; olefin polymers such as polyethylene, polypropylene and the like; and these resins may be a copolymer or a crosslinked polymer. In the present invention, the composite resin particle in which the above-mentioned polymers are arbitrarily combined is used depending on the use and the purpose of the cosmetic.

Inter alia, from a viewpoint of excellence in retention of the effect of the cosmetic, a composite resin particle in which a recess part consists of an aromatic vinyl polymer and a particle body consists of (meth)acrylic acid ester polymer, and a composite resin particle in which a recess part and a particle body consist of different kinds of (meth)acrylic acid ester polymers are preferably used. Especially, a composite resin particle consisting of polystyrene and polymethyl methacrylate, and a composite resin particle consisting of polymethyl methacrylate, and one kind selecting from polymethacrylic acid alkyl esters having an alkyl group of a carbon number of 2-18 are preferably used. (In the present specification, "(meth)acrylic" means "acrylic" or " methacrylic".)

Examples of a process for producing the spherical resin particle having many recesses on the surface are not limited to, but include a seed dispersion polymerization method, and a seed emulsion polymerization method, and the seed dispersion polymerization method is particularly preferable. In the seed dispersion polymerization method, in the case of the composite resin in which a recess part consists of an aromatic vinyl polymer and a particle body consists of an (meth)acrylic acid ester polymer, a spherical resin particle having many recesses on the surface can be obtained by a method of seed dispersion-polymerizing an aromatic vinyl polymer in a solvent (S1) in which the spherical particles of an (meth)acrylic acid ester polymer are dispersed as a seed particle, isolating particles from the milky lotion-like reaction mixture by centrifugal separation or the like, and drying them. In this case, it is preferable that the aromatic vinyl polymer which is to be a recess part is a polymer of a monomer which is soluble in the solvent (S1) and has lower or equivalent affinity for the solvent (S1) as compared with that of the seed particles. For example, as the solvent (S1), a methanol/water mixed solvent is used. It is preferable that the polymerization reaction is carried out in the presence of a polymerization initiator, a dispersant, and an organic solvent (S2) which is a poor solvent or a nonsolvent of a (meth)acrylic acid ester polymer constituting seed particles, but is a good solvent of an aromatic vinyl polymer, and is insoluble or partially soluble in the solvent (S1). For example, as the organic solvent (S2), decalin is preferably used.

Alternatively, the spherical resin particle having many recesses on the surface used in the present invention may be obtained, for example, by a method of dispersing spherical particles of an aromatic vinyl polymer such as polystyrene obtained by the known emulsion polymerization method as a seed particle into a dispersion solvent such as an ethanol/water mixed solvent, mixing the dispersion with (meth)acrylic acid ester such as butyl acrylate, allowing the mixture to stand at 0°C to obtain swelling particles, seed emulsion-polymerizing them, isolating particles from a milky lotion-like reaction mixture by centrifugal separation or the like, and drying them.

Since the spherical resin particle obtained by the above-mentioned method has many recesses which are regularly arranged on the surface, and forms a composite structure in which a recess part and a particle body are constructed of different kinds of polymers, a cosmetic which exhibits the homogeneous effect due to selective absorption and maintenance in the recess part of an active ingredient having high affinity for a polymer of the recess part, when blended in the cosmetic, and which is excellent in retention of the effect, can be provided.

Furthermore, a spherical resin particle which carries an oleophilic component in the recess part can be obtained by a method of dispersing the spherical resin particle obtained by the above-mentioned method in a medium in which the oleophilic component to be blended into the cosmetic is dissolved therein, selectively absorbing the component into the recess part to be carried therein, isolating the particles by centrifugal separation or the like, and washing them with a poor solvent which hardly dissolves the oleophilic component, followed by drying.

Since the thus obtained spherical resin particle carries the oleophilic component at a high concentration only in the recess part on the surface, a cosmetic which is more excellent in retention of the effect and is excellent in a use feeling can be obtained when blended the spherical particle in the cosmetic.

The oleophilic component which is carried in the recess part on the surface can be arbitrarily selected as far as it has high skin safety, which is generally used in the cosmetic. Examples of the oleophilic component include higher alcohols such as lauryl alcohol, stearyl alcohol, oleyl alcohol, palmityl alcohol, lanolin alcohol, isostearyl alcohol and the like; higher fatty acids such as lauric acid, myristic acid, palmitic acid, stearic acid, behenic acid, oleic acid, 12-hydroxystearic acid, linolenic acid, linoleic acid and the like; higher fatty acid esters such as isopropyl myristate, isopropyl palmitate, cetyl 2-ethylhexanoate, butyl stearate and the like; hydrocarbons such as liquid paraffin, squalane, Vaseline and the like; polyhydric alcohols such as propylene glycol, butylene glycol and the like. In addition, depending on the use and the purpose, a surfactant, a perfume, an antimicrobial agent, an antiseptic/germicide agent, an antiphlogistic agent, an ultraviolet absorber, an antioxidant and the like may be contained.

Examples of the cosmetic in the present invention include makeup cosmetics such as face powder, foundation, lip stick, lip cream, rouge, eyeliner, eye shadow, mascara and the like; body cosmetics such as body powder, body lotion and the like; skin care products such as lotion, cream, milky lotion and the like; hair cosmetics such as shampoos, rinse, hair treatment, hair mousse, hair coloring and the like; antiperspirants; sunscreen agents; and the like. Especially, the cosmetic of the invention is preferably a makeup cosmetic, from a viewpoint that it has a characteristic optical property such as high light-scattering property since the particle used has many recesses on the surface and, from a viewpoint that, when blended in the cosmetic, the optical function can be imparted, such as color-rendering impartation and improvement in a foggy feeling (the wrinkle degradation effect). Moreover, from a viewpoint that the particle used carries an oleophilic component such as an emollient component which imparts softness and smoothness to a skin in the recess part, thereby, retention of the effect can be improved, a skin care product is preferable.
Furthermore, since the particle used selectively adsorbs sebum in the recess part, thereby, can suppress a sticky feeling due to sweating, and can retain a dry feeling for a long time, an antiperspirant can be also preferably used.

A ratio of the spherical resin particle having many recesses on the surface to be blended in the cosmetic of the present invention is not particularly limited, but is desirably 1 to 30%, preferably 3 to 20% by weight based on a total amount of the cosmetic. When the blending ratio of the particle is less than 1% by weight, there is a possibility that the effect excellent in spreadability, and a touch feeling at application can not be fully exerted. On the other hand, when the blending ratio exceeds 30% by weight, there is a possibility that it is not economical and a touch feeling at application is conversely deteriorated.

### Examples

Preparation Examples, Eexamples, and Comparative Examples will be especially explained below, but the present invention is not limited to these Examples.

### Preparation Example 1

A reaction vessel of 2L volume equipped with a stirrer and a condenser was charged with 504g of methanol, 216g of pure water, and 54g of decalin, 1.8g of polyvinyl pyrrolidone as a dispersant was dissolved therein, and 30g of polymethyl methacrylate spherical particles (2 µm of volume average particle diameter, 2.5% of CV value) were added as a seed particle to be dispersed therein.

Furthermore, after 18g of styrene as a monomer and 1.2g of azobisisobutyronitril as a polymerization initiator were dissolved and then the inside of a reaction vessel was brought into the nitrogen atmosphere, a polymerization reaction was completed by rising a temperature and maintaining a temperature in the reaction system at 60°C for 24hr.

The resultant milky lotion-like reaction mixture was subjected to solid liquid separation by centrifugal separation, the isolated resin particles were washed with methanol, and dried at 25°C under reduced pressure to obtain 46.5g of spherical resin particles having a volume average particle diameter of 2.2 µm, a CV value of 2.6%, an average diameter of an opening of 0.1 µm, and the average number of recesses per particle of 120.

### Preparation Example 2

According to the same manner as that of Preparation Example 1 except that 13g of decalin was used, 47.1g of spherical resin particles were obtained. The resultant particles had a volume average particle diameter of 2.1 µm, a CV value of 2.7%, an average diameter of an opening of 0.03 µm, and the average number of recesses per particle of 350.

### Preparation Example 3

According to the same manner as that of Preparation Example 1 except that 36g of styrene was used, 62.4g of spherical resin particles were obtained. The resultant particles had a volume average particle diameter of 2.2 µm, a CV value of 2.7%, an average diameter of an opening of 0.3 µm, and the average number of recesses per particle of 50.

### Preparation Example 4

According to the same manner as that of Preparation Example 1 except that polymethyl methacrylate spherical particles having a volume average particle diameter of 10 µm (a CV value of 7.1%) were used as a seed particle, 46.2g of spherical resin particles were obtained. The resultant particles had a volume average particle diameter of 10.1 µm, a CV value of 7.2%, an average diameter of an opening of 0.05 µm, and the average number of recesses per particle of 2400.

### Preparation Example 5

According to the same manner as that of Preparation Example 3 except that polymethyl methacrylate spherical particles having a volume average particle diameter of 50 µm (a CV value of 16.4%) were used as a seed particle, 61.3g of spherical resin particles were obtained. The resultant particles had a volume average particle diameter of 50.0 µm, a CV value of 16.5%, an average diameter of an opening of 0.05 µm, and the average number of recesses per particle of 2600.

### Preparation Example 6

Five parts by weight of the spherical resin particles obtained in Preparation Example 4 were uniformly dispersed in 100 parts by weight of squalane, the dispersion was maintained at 20-25°C for 24 hrs, and particles were isolated by centrifugal separation.
Furthermore, the resultant particles were washed with 100 parts by weights of normal hexane, isolated again by centrifugal separation and dried at 25°C to obtain spherical resin particles carring squalane in the recess part.

### Preparation Example 7

Styrene was emulsion-polymerized at 70°C in the absence of an emulsifier in a water/ethanol (25/75 weight ratio) mixed solution under the nitrogen atmosphere to obtain an emulsion of polystyrene particles.

This emulsion corresponding to 70g of a resin solid content was placed into a reaction vessel of 500ml volume equipped with a stirrer and a condenser, mixed with 30g of butyl acrylate, and allowed to stand at 0°C for 24 hrs to absorb butyl acrylate into polystyrene particles.

After 0.6g of potassium persulfate was added and a composition of a water/ethanol mixed solution was adjusted to 90/10, polymerization was performed at 70°C for 24 hrs to obtain spherical resin particles having a volume average particle diameter of 0.6 µm, a CV value of 2.1%, an average diameter of an opening of 0.08 µm, and the average number of recesses per particle of 40.

### Preparation Example 8

According to the same manner as that of Preparation Example 1 except that polymethyl methacrylate spherical particles having a volume average particle diameter of 10 µm (a CV value of 7.1%) were used as a seed particle and cyclohexyl methacrylate was used as a monomer, 46.0g of spherical resin particles were obtained. The resultant particles had a volume average particle diameter of 10.3 µm, a CV value of 8.1%, an average diameter of an opening of 0.91 µm, and the average number of recesses per particle of 150.

### Preparation Example 9

According to the same manner as that of Preparation Example 1 except that polymethyl methacrylate spherical particles having a volume average particle diameter of 10 µm (a CV value of 7.1%) were used as a seed particle and stearyl methacrylate was used as a monomer, 45.8g of spherical resin particles were obtained. The resultant particles had a volume average particle diameter of 10.5 µm, a CV value of 10.2%, an average diameter of an opening of 0.95 µm, and the average number of recesses per particle of 130.

### Preparation Example 10

According to the same manner as that of Preparation Example 6 except that the spherical resin particles obtained in Preparation Example 8 were used, spherical resin particles carring squalane in the recess part were obtained.

### Example 1

A powder consisting of 15% by weight of the spherical resin particles having many recesses on the surface, obtained in Preparation Example 1, 8% by weight of titanium oxide, 45.2% by weight of sericite, 10% by weight of talc, 2% by weight of mica-titanium, 2% by weight of red oxide, 3.5% by weight of yellow iron oxide, 1% by weight of ultramarine blue, 1% by weight of aluminum stearate, 5% by weight of dimethylpolysiloxane, 7% by weight of squalane, 0.2% by weight of paraben, and 0.1% by weight of perfume, was mixed by using a Henschel mixer, and an oil was added thereto. This mixture was filled in a vessel to obtain a solid foundation.

### Examples 2-7

According to the same manner as that of Example 1 except that the spherical resin particles obtained in any one of Preparation Examples 2-5 and 8-9 shown in Table 1 were used instead of the spherical resin particles obtained in Preparation Example 1, a solid foundation was obtained.

### Examples 8-9

According to the same manner as that of Example 1 except that the spherical resin particles carring squalane in the recess part, obtained in any one of Preparation Examples 6 and 10 were used instead of the spherical resin particles obtained in Preparation Example 1, a solid foundation was obtained.

### Comparative Example 1

According to the same manner as that of Example 1 except that the polymethyl methacrylate spherical particles having no recesses on the surface, and having a volume average particle diameter of 2.0 µm, and a CV value of 2.5% were used instead of the spherical resin particles obtained in Preparation Example 1, a solid foundation was obtained.

### Comparative Example 2

According to the same manner as that of Example 1 except that the polymethyl methacrylate spherical particles having no recesses on the surface, and having a volume average particle diameter of 10.0 µm, and a CV value of 7.1% were used instead of the spherical resin particles obtained in Preparation Example 1, a solid foundation was obtained.

### Comparative Example 3

According to the same manner as that of Example 1 except that the polymethyl methacrylate spherical particles having no recesses on the surface, and having a volume average particle diameter of 50.0 µm, and a CV value of 16.4% were used instead of the spherical resin particles obtained in Preparation Example 1, a solid foundation was obtained.

### Example 10

1.2 parts by weight of polyoxyethylene sorbitan monostearate, 1.3 parts by weight of polyoxy sorbitol tetraoleate, 1.3 parts by weight of glyceryl monostearate, 0.5 part by weight of stearic acid, 0.9 part by weight of biphenyl alcohol, 0.5 part by weight of cetyl parmitate, 5 parts by weight of squalane, and 4 parts by weight of cetyl 2-ethylhexenate, and 0.5 part by weight of polymethylsiloxane were warmed to 80°C or higher, followed by mixing (Solution A). Similarly, 10 parts by weight of 1,3-butylene glycol, 0.2 part by weight of xanthan gum, and 69.2 parts by weight of purified water were warmed to 80°C or higher, followed by mixing (Solution B).

The resulting solutions A and B were mixed gradually while warming to 80°C or higher, and subsequently cooled to 50°C, the spherical resin particles having many recesses on the surface obtained in Preparation Example 1 were added to 5% by weight relative to a total weight and the mixture was further mixed while stirring to obtain a milky lotion.

### Examples 11-15

According to the same manner as that of Example 10 except that the spherical resin particles obtained in any one of Preparation Examples 2-4 and 8-9 shown in Table 2 were used instead of the spherical resin particles obtained in Preparation Example 1, a milky lotion was obtained.

### Examples 16-17

According to the same manner as that of Example 10 except that the spherical resin particles carring squalane in the recess part, obtained in any one of Preparation Examples 6 and 10 were used instead of the spherical resin particles obtained in Preparation Example 1, a milky lotion was obtained.

### Example 18

According to the same manner as that of Example 10 except that the spherical resin particles having many recesses on the surface, obtained in Preparation Examples 7 were used instead of the spherical resin particles obtained in Preparation Example 1, a milky lotion was obtained.

### Comparative Example 4

According to the same manner as that of Example 10 except that the polymethyl methacrylate spherical particles having no recesses on the surface, and having a volume average particle diameter of 2.0 µm, and a CV value of 2.5% were used instead of the spherical resin particles obtained in Preparation Example 1, a milky lotion was obtained.

### Comparative Example 5

According to the same manner as that of Example 10 except that the polymethyl methacrylate spherical particles having no recesses on the surface, and having a volume average particle diameter of 10.0 µm, and a CV value of 7.1% were used instead of the spherical resin particles obtained in Preparation Example 1, a milky lotion was obtained.

### Example 19

To the mixed solution of 64 parts by weight of ethanol, 2 parts by weight of propylene glycol, 0.5 part by weight of a perfume, and 13.5 parts by weight of purified water, were added 15 parts by weight of basic aluminum chloride and 5 parts by weight of the spherical resin particle having many recesses on the surface obtained in Preparation Example 1, and the mixture was mixed while stirring to obtain an antiperspirant.

### Example 20

According to the same manner as that of Example 19 except that the spherical resin particles obtained in Preparation Example 4 were used instead of the spherical resin particles obtained in Preparation Example 1, an antiperspirant was obtained.

### Examples 21-23

According to the same manner as that of Example 19 except that the spherical resin particles obtained in Preparation Examples 5 and 8-9 were used instead of the spherical resin particles obtained in Preparation Example 1, an antiperspirant was obtained.

### Comparative Example 6

According to the same manner as that of Example 19 except that the polymethyl methacrylate spherical particles having no recesses on the surface, and having a volume average particle diameter of 2.0 µm, and a CV value of 2.5% were used instead of the spherical resin particles obtained in Preparation Example 1, an antiperspirant was obtained.

### Comparative Example 7

According to the same manner as that of Example 19 except that the polymethyl methacrylate spherical particles having no recesses on the surface, and having a volume average particle diameter of 10.0 µm, and a CV value of 7.1% were used instead of the spherical resin particles obtained in Preparation Example 1, an antiperspirant was obtained.

### Comparative Example 8

According to the same manner as that of Example 19 except that the polymethyl methacrylate spherical particles having no recesses on the surface, and having a volume average particle diameter of 50.0 µm, and a CV value of 16.4% were used instead of the spherical resin particles obtained in Preparation Example 1, an antiperspirant was obtained.

### Assessment

A use feeling of the solid foundation, the lotion and the antiperspirant obtained in each of Examples and Comparative Examples, was assessed based on the following Assessment Criteria by applying them to skins of 10 assessment panelists, and summing the number of panelists who had felt good in an extent of spreadability and a touch feeling (no foreign-body sensation) at application, and a moist feeling and retention thereof for the solid foundation or lotion, and an extent of a dry feeling and retention thereof for the antiperspirant. The results are shown in Tables 1-3.

### Evaluation Criteria

⊕: Excellent (The number of persons who sensed that it is good, is 10)
o: Good (The number of persons who sensed that it is good, is 8-9)
Δ: Slightly bad (The number of persons who sensed that it is good, is 6-7)
x : Bad (The number of persons who sensed that it is good, is not more than 5)

**Table 1**

| | Spherical resin particle | Volume average particle diameter [µm] | Average diameter of opening [µm] | Spread-ability | Touch feeling | Moist feeling | Retention of moist feeling |
|---|---|---|---|---|---|---|---|
| Example 1 | Preparation Example 1 | 2.2 | 0.1 | ○ | ⊕ | ⊕ | ○ |
| Example 2 | Preparation Example 2 | 2.1 | 0.03 | ○ | ⊕ | ⊕ | ○ |
| Example 3 | Preparation Example 3 | 2.2 | 0.3 | ○ | ⊕ | ⊕ | ○ |
| Example 4 | Preparation Example 4 | 10.1 | 0.05 | ⊕ | ⊕ | ⊕ | ○ |
| Example 5 | Preparation Example 5 | 50.0 | 0.05 | ⊕ | ○ | ○ | ○ |
| Example 6 | Preparation Example 8 | 10.3 | 0.91 | ⊕ | ⊕ | ⊕ | ○ |
| Example 7 | Preparation Example 9 | 10.5 | 0.95 | ⊕ | ⊕ | ⊕ | ○ |
| Example 8 | Preparation Example 6 | 10.1 | 0.05 | ⊕ | ⊕ | ⊕ | ⊕ |
| Example 9 | Preparation Example 10 | 10.3 | 0.91 | ⊕ | ⊕ | ⊕ | ⊕ |
| Comparative Example 1 | PMMA | 2.0 | - | ○ | ⊕ | △ | △ |
| Comparative Example 2 | PMMA | 10.0 | - | ⊕ | ⊕ | △ | x |
| Comparative Example 3 | PMMA | 50.0 | - | ⊕ | ○ | △ | x |

**Table 2**

| | Spherical resin particle | Volume average particle diameter [µm] | Average diameter of opening [µm] | Spread-ability | Touch feeling | Moist feeling | Retention of moist feeling |
|---|---|---|---|---|---|---|---|
| Example 10 | Preparation Example 1 | 2.2 | 0.1 | ○ | ⊕ | ⊕ | ○ |
| Example 11 | Preparation Example 2 | 2.1 | 0.03 | ○ | ⊕ | ⊕ | ○ |
| Example 12 | Preparation Example 3 | 2.2 | 0.3 | ○ | ⊕ | ⊕ | ○ |
| Example 13 | Preparation Example 4 | 10.1 | 0.05 | ⊕ | ⊕ | ⊕ | ○ |
| Example 14 | Preparation Example 8 | 10.3 | 0.91 | ⊕ | ⊕ | ⊕ | ○ |
| Example 15 | Preparation Example 9 | 10.5 | 0.95 | ⊕ | ⊕ | ⊕ | ○ |
| Example 16 | Preparation Example 6 | 10.1 | 0.05 | ⊕ | ⊕ | ⊕ | ⊕ |
| Example 17 | Preparation Example 10 | 10.3 | 0.91 | ⊕ | ⊕ | ⊕ | ⊕ |
| Example 18 | Preparation Example 7 | 0.6 | 0.08 | ○ | ⊕ | ⊕ | ○ |
| Comparative Example 4 | PMMA | 2.0 | - | ○ | ⊕ | △ | △ |
| Comparative Example 5 | PMMA | 10.0 | - | ⊕ | ⊕ | △ | x |

**Table 3**

| | Spherical resin particle | Volume average particle diameter [µm] | Average diameter of opening [µm] | Dry feeling | Retention of dry feeling |
|---|---|---|---|---|---|
| Example 19 | Preparation Example 1 | 2.2 | 0.1 | ○ | ⊕ |
| Example 20 | Preparation Example 4 | 10.1 | 0.05 | ⊕ | ⊕ |
| Example 21 | Preparation Example 5 | 50.0 | 0.05 | ⊕ | ○ |
| Example 22 | Preparation Example 8 | 10.3 | 0.91 | ⊕ | ⊕ |
| Example 23 | Preparation Example 9 | 10.5 | 0.95 | ⊕ | ⊕ |
| Comparative Example 6 | PMMA | 2.0 | - | ○ | x |
| Comparative Example 7 | PMMA | 10.0 | - | ⊕ | x |
| Comparative Example 8 | PMMA | 50.0 | - | ⊕ | x |

Tables 1 and 2 show that the cosmetics of Examples 1-18 comprising the spherical resin particle having many recesses on the surface, are excellent in the levels of spreadability, a touch feeling, and a moist feeling, as well as retention thereof. On the other hand, it is recognized that the cosmetics of Comparative Examples 1-5, comprising the polymethyl methacrylate spherical particle having no recess on the surface, are excellent in spreadability and a touch feeling, but inferior in a moist feeling and retention thereof. From Table 3, it is recognized that the antiperspirants of Comparative Examples 6-8 are excellent in a dry feeling, but inferior in retention of dry feeling.

### Industrial Applicability

Since the cosmetic of the present invention is excellent in spreadability and a touch feeling at application, and an effect after application, as well as retention thereof, the cosmetic can be suitably utilized for foundation, lip stick, rouge, eyeliner, eye shadow, eyebrow, mascara, face powder, dusting powder, cream, lotion, preshave lotion, aftershave lotion, milky lotion, moisture lotion, antiperspirant and the like.

## Claims

1. A cosmetic comprising a spherical resin particle having many recesses on the surface thereof.

2. The cosmetic according to claim 1, wherein a volume average particle diameter of the particle is 0.1 to 50 µm.

3. The cosmetic according to claim 1 or 2, wherein an average diameter of an opening of the recesses is 0.01 to 1 µm.

4. The cosmetic according to any one of claims 1 to 3, wherein a particle size distribution of the spherical resin particle is not more than 20% as expressed by a variation coefficient CV value.

5. The cosmetic according to any one of claims 1 to 4, wherein the spherical resin particle is a composite resin particle in which a recess part and a particle body are composed of different resins.

6. The cosmetic according to any one of claims 1 to 5, wherein the spherical resin particle is a composite resin particle consisting of an aromatic vinyl polymer and a (meth)acrylic acid ester polymer.

7. The cosmetic according to claim 6, wherein the aromatic vinyl polymer is polystyrene and the (meth)acrylic acid ester polymer is polymethyl methacrylate.

8. The cosmetic according to any one of claims 1 to 5, wherein the spherical resin particle is a composite resin particle consisting of different kinds of (meth)acrylic acid ester polymers.

9. The cosmetic according to claim 8, wherein the different kinds of (meth)acrylic acid ester polymers are one kind of those selected from polymethacrylic acid alkyl esters having an alkyl group of a carbon number of 2-18, and polymethyl methacrylate.

10. The cosmetic according to any one of claims 1 to 9, wherein the spherical resin particle is a particle obtained by a seed dispersion polymerization method.

11. The cosmetic according to any one of claims 1 to 10, wherein the spherical resin particle is a particle which carries an oleophilic component in the recess part.

12. The cosmetic according to any one of claims 1 to 11, wherein the cosmetic is a makeup cosmetic.

13. The cosmetic according to any one of claims 1 to 11, wherein the cosmetic is a skin care product.
